# EUROPEAN PATENT APPLICATION

(11) **EP 4 663 763 A2**
(43) Date of publication of application: **17.12.2025**
(21) Application number: 25196865.7
(22) Date of filing: 23.05.2019
(51) Int. Cl.: C12N 15/87

(54) **METHODS TO IDENTIFY STRUCTURAL VARIATIONS THAT CAUSE DISEASES AND THE REGIONS TO REPAIR WITH GENE EDITING**

(30) Priority: 23.05.2018 US 201862675486 P; 17.01.2019 US 201962793793 P
(62) Divisional of application: 19807825.5
(71) Applicant: Williwaw Biosciences LLC, Clarkston, Michigan 48348 (US)
(72) Inventor: GARVIN, Michael, Clarkston, 48348 (US)
(74) Representative: Dehns

(57) **Abstract**

Methods of identifying a genetic structural variation include assembling single nucleotide polymorphism (SNP) data from parents and their offspring; analyzing the SNP data for at least one non-Mendelian inheritance pattern (NMI), filtering to remove normal structural variation, and filtering to identify regions that should be conserved but are disrupted. The disrupted regions may be specific to a disease or disorder of interest. Methods of identifying a structural variation in a genome of a patient and treating a disease or disorder related to the structural variation are also disclosed.

## Description

### CROSS REFERENCE TO RELATED APPLICATIONS

This application claims priority to U.S. Provisional Patent Application No. 62/675,486, filed May 23, 2018 and titled "GENETIC AND POSSIBLE MECHANISTIC BASIS OF AUTISM SPECTRUM DISORDER," and to U.S. Provisional Patent Application No. 62/793,793, filed January 17, 2019 and titled "METHOD TO IDENTIFY GENETIC CAUSES OF HUMAN DISEASE," and the contents of each are hereby incorporated by reference in their entirety.

### TECHNICAL FIELD

This disclosure generally relates to methods of identifying structural variations that correlate with or cause diseases or disorders.

### BACKGROUND

Many major human diseases and disorders have a genetic component, but current methods such as Genome Wide Association Studies (GWAS) have failed to identify highly correlative, let alone causative, genetic changes. For example, the heritability of Autism Spectrum Disorder (ASD) has been estimated to be between 60% and 90%, but two independent GWAS were only able to identify single nucleotide polymorphisms (SNPs) that explained a handful of cases, and these loci have not been replicated in other studies. More recent work with significantly larger sample sizes achieved comparable results, and studies of other neurodevelopmental disorders such as Bipolar Disorder (BPD) and Attention Deficit Hyperactivity Disorder (ADHD) have not identified significant correlative or causative markers either. Similarly, the overall risk of developing Multiple Sclerosis (MS) is roughly 1 in 1,000 and increases to 1 in 4 in identical twins, emphasizing the genetic component of the disease. More than 200 genes have been identified as risk factors but no causative genetic change(s) are known, and the 200 genes explain only a small percentage of the cases. A recent study on Major Depression using the UK Biobank data from more than 400,0000 individuals was unable to replicate a single previously reported candidate locus for that trait. The authors suggest that all previously reported significant SNP loci are false positives due to lack of statistical power. It should be noted that the heritability of Major Depression, BPD, and ADHD are similar to ASD.

A need remains for accurately identifying correlative and causative genetic changes in human diseases and disorders.

### SUMMARY

Methods of identifying genetic structural variations are disclosed herein. In one implementation, a method of identifying a structural variation in a genome includes assembling single nucleotide polymorphism (SNP) data from parents and their offspring. The SNP data is analyzed for at least one non-Mendelian inheritance pattern (NMI). The NMI is a potential structural variation. The NMI are scored to identify large structural variations from sequential SNPs that demonstrate NMI in the offspring. SNPs are removed that demonstrate NMI in the offspring but that overlap with known existing variation. Conserved regions of the genome are identified to filter regions that should be conserved but include a structural variation. Potentially biologically important structural variation is screened for.

In additional or alternative embodiments, NMI of high frequency in a population may be verified to determine if the NMI of high frequency are structural variations. SNPs of NMI that are structural variations but that overlap with known existing variation may be removed. High frequency may be an occurrence of greater than 5% in the population.

In additional or alternative embodiments, the potentially biologically important structural variation may be one or more of a structural variation that resides in a gene in which less than 5% of normal individuals have a known structural variation; there is a custom correlation coefficient bloc in the gene; a frequency of the NMI at one site in the gene is greater than 5% in a diseased population; and there is a run of at least four SNPs with NMI in a row. Conserved regions of the genome may be identified by a custom correlation coefficient (CCC) analysis. A probability on having a run of NMI may be assigned and SNPs with a run of NMI greater than 4 may be maintained. NMI attributable to high levels of masked repetitive elements may be removed. Pinpoint locations of both the structural variations and conserved blocs of genetic information may be identified. The locations of the structural variations and the conserved blocs of genetic information may be used to identify locations of rare structural variations in genes that have conserved blocs of genetic information.

In another implementation, a method of identifying a structural variation in a genome of a patient and then treating a disease or disorder related to the structural variation includes assembling single nucleotide polymorphism (SNP) data from parents and their offspring. The SNP data is analyzed for at least one non-Mendelian inheritance pattern (NMI). The NMI is a potential structural variation. The NMI are scored to identify large structural variations from sequential SNPs that demonstrate NMI in the offspring. SNPs are removed that demonstrate NMI in the offspring but that overlap with known existing variation. Conserved regions of the genome are identified to filter regions that should be conserved but include a structural variation. Structural variation related to the disease or disorder is screened for. The patient is identified as having the structural variation related to the disease or disorder and is treated.

In additional or alternative embodiments, NMI of high frequency in a population may be verified to determine if the NMI of high frequency are structural variations. SNPs of NMI that are structural variations but that overlap with known existing variation may be removed. High frequency may be an occurrence of greater than 5% in the population.

In additional or alternative embodiments, the structural variation related to the disease or disorder may be selected from one or more of a structural variation that resides in a gene in which less than 5% of normal individuals have a known structural variation; there is a custom correlation coefficient bloc in the gene; a frequency of the NMI at one site in the gene is greater than 5% in a diseased population; and there is a run of at least four SNPs with NMI in a row. Conserved regions of the genome may be identified by a custom correlation coefficient (CCC) analysis. Structural variations that are not specific to the disease or disorder may be filtered out. Treating the patient may include a gene editing technology. The gene editing technology may include CRISPR. Treating the patient may include administration of CAR T cells.

### BRIEF DESCRIPTION OF THE DRAWINGS

Figure 1A is a flow diagram of a structural variation identification method according to one embodiment.
Figure 1B is a flow diagram of a method of identifying structural variation and treating a disease or disorder related to the structural variation.
Figure 2A is an illustration of a cryptic genetic variation (CGV) in one family.
Figure 2B is an illustration of the pattern of Non-Mendelian Inheritance (NMI) most often seen in data, which is either inherited or *de novo.*
Figure 2C is an illustration of non-allelic recombination-repair resulting in CGV.
Figure 2D is an illustration of a null allele caused by INDELS that disrupt the binding of a genotyping probe.
Figure 3A is an illustration of a portion of the NRXN3 gene including the SNP rs221465. An 8.6kb deletion at this site with an allele frequency of 0.28, according to the 1000 Genome data, is also shown.
Figure 3B is a genotype plot from ASD children of the SNP of Figure 3A.
Figure 4 shows properties of NMI loci. Figure 4A is a graph of the mean number of NMI loci per individual for children from Simplex families and from Multiplex families. Figure 4B is a graph of the number of genes with NMI in the SFARI data set and in reported differentially expressed genes in post mortem brain tissue of ASD individuals. Figure 4C is a graph of the ratio of observed to expected overlap between genes in which SNPs reside and transposable elements. Figure 4D is a graph of the relationship between NMI and gene length.
Figure 5 is an illustration of layered analyses of detection of structural variation (SV), presence of a CCC block of SNPs (CCC), and differential gene expression in ASD brain (DEG).
Figures 6A and 6B are graphs of the number of significant to non-significant, or vice versa, LD changes in the ST6GALNAC5 locus surrounding SNP rs12084236 (Figure 6A) or in the ANKRD28 locus surrounding SNP rs12084236 (Figure 6B). Figure 6C is a plot of heterozygosity to verify SV.
Figure 7 illustrates a mechanism of SV generation using CTNNA3 as an example.

### DETAILED DESCRIPTION

Early evaluations of the failure of Genome Wide Association Studies (GWAS) to identify causal or highly associated variants for diseases and phenotypic traits pointed to "missing data" or cryptic genetic variation (CGV) such as copy number variation (CNV) or other structural variants (SV). CGV and SV may be used interchangeably herein to describe genomic variation larger than one single nucleotide polymorphism (SNP) as a possible cause. Subsequent work assumed that the missing data was simply a *lack* of data, *i.e.* the missing data was the realization that many thousands of SNPs of small effect produce variation in phenotypic traits or cause disease rather than a few genetic variants with large effects. As evidence of this, researchers have demonstrated that with larger sample sizes and more genetic information, larger numbers of associated SNPs are found for a trait. However, following this logic, nearly 100,000 common SNPs of tiny effect are responsible for determining height; this translates into a trait-effecting SNP in each 100kb block of the human genome.

Alternatively, it is possible that the original hypothesis was correct; there are missing data of large effect in the form of CGV that can explain human diseases and disorders but they were simply not detected previously, or were not included in the analyses. It has become increasingly clear that CGV represents a biologically important source of genetic variation for evolution and adaptation to local environments in many species, and having them can increase the risk of developing disorders such as Autism Spectrum Disorder (ASD), but it is often unclear if or how a specific CGV does so; a major reason is that CGV is, as the name implies, cryptic. The enigmatic nature of the variation is due in part to the inability of widely used short-read sequencing technologies such as Illumina to detect CGVs. This is because Illumina and other sequencing approaches rely on accurately mapping reads from the output onto a reference genome. If the reference genome does not contain accurate information (e.g. a stretch of sequence is inserted or deleted), the reads will not be mapped and they will be discarded. With newer long-read technology, the CGV needs to occur on the same contiguous region of DNA (e.g. transposed DNA fragments cannot be identified). As with short-read technology, long-read also relies on an accurate reference genome. Finally, technology such as hybridization arrays query only known CGV so any de novo genetic alterations will be missed. In sum, presently available technologies in the art cannot accurately identify CGV.

Studies on individuals with neurodevelopmental disorders such as ASD are also compromised because they are phenotypically heterogeneous and it is likely the variation in subtypes is the result of mutations in different biological pathways or that similar phenotypes are the result of disruptions in different genes within a single biochemical network. This may also be true for diseases like multiple sclerosis (MS), which presents as many sub-types. Therefore, any test group of cases will be comprised of genetically different subgroups, resulting in a loss of statistical power. Individuals in control groups may harbor partial molecular disease networks and have a mild form of the disorder, which again compromises statistical robustness. Furthermore, epistasis (multiple alleles interacting to produce a trait) is likely a component of complex phenotypes such as neurodevelopmental disorders, which reduces the power of single-SNP tests such as standard GWAS approaches. Finally, the causative disruptions may be in non-coding regions of the genome, decreasing the effectiveness of Whole Exome Sequencing (WES) approaches that are currently the focus of many studies.

A simple, inexpensive process to identify cryptic variation in the genome of any sexually reproducing species using non-Mendelian inheritance patterns and the CCC approach from SNP-based genomic data is disclosed herein. The process may include documenting all structural variation (SV) within a single individual. The SV may be tested for association with any trait of interest, including a disease or disorder. The exact location of the SV may be pinpointed and repaired with gene editing technology such as CRISPR, using the homologous chromosome as a guide for with what sequence to replace the SV. In gene editing, somatic cells but not germline cells may be altered, which may limit the effect of the editing to the patient and not affect any future offspring.

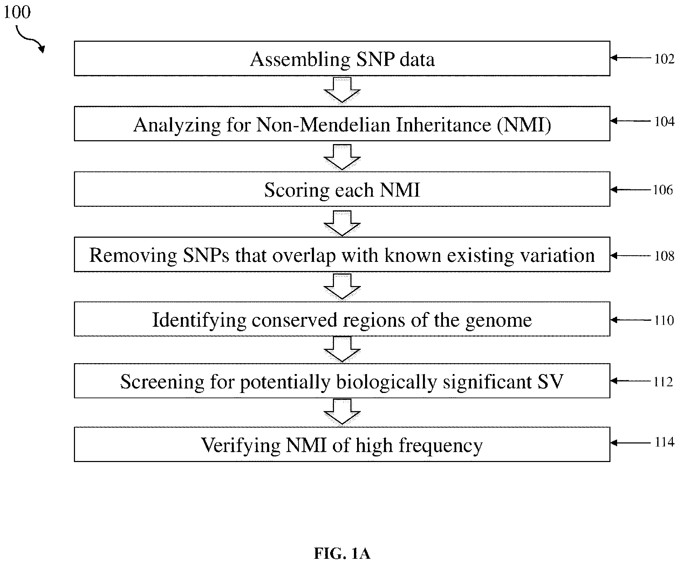

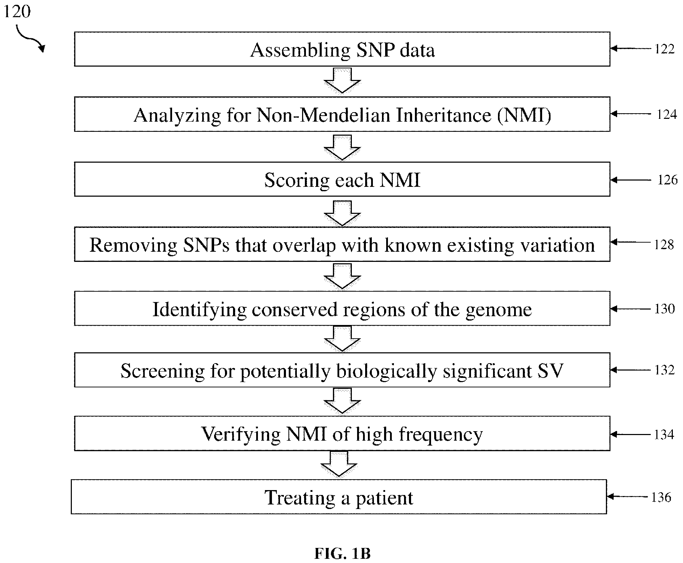

The present disclosure includes methods to identify biologically relevant CGV for a given disorder using parent-child SNP genotypes (see Figure 1). In some implementations, the methods query an individual's genome for regions that are meant to be conserved but have been disrupted by CGV. Conserved regions may be biologically important regions. In some examples, data from individuals with ASD or MS were used to identify causative genes and biochemical pathways that may help explain each of those disorders. The state of art can explain less than 5% of cases of ASD or MS. In contrast, the presently disclosed methods explain nearly every case of ASD and MS. The state of art, such as known sequencing methods, cannot accurately and reliably pinpoint CGV locations. In contrast, the presently disclosed methods may pinpoint the location of a CGV within a gene. Such accurate identification may help provide specific locations that can be altered with gene editing technology such as CRISPR. Gene editing technologies may be used to treat the disease or disorder that has been diagnosed or identified using the methods disclosed herein.

In one embodiment, and with reference to Figure 1A, a method 100 of identifying a genetic structural variation is disclosed. The method 100 may include assembling SNP data 102; analyzing for non-Mendelian inheritance (NMI) 104; scoring each NMI 106; removing SNPs that overlap with known existing variation 108; identifying conserved regions of the genome 110; screening for potentially biologically important SV 112; and verifying NMI of high frequency 114.

Each of assembling SNP data 102; analyzing for non-Mendelian inheritance (NMI) 104; scoring each NMI 106; removing SNPs that overlap with known existing variation 108; identifying conserved regions of the genome 110; screening for potentially biologically important SV 112; and verifying NMI of high frequency 114 may be performed in any order, including concurrently.

The SNP data in assembling SNP data 102 may be SNP data from parents and their offspring. The SNP data may be sourced as described in Example 1.

Analyzing NMI 104 may be performed as described in Example 1. All NMI may be considered potential SV.

Scoring each NMI 106 may include identifying large structural variations from sequential SNPs that demonstrate NMI in the offspring. The NMI may be scored as "1." Scoring 106 may also include sorting each NMI by chromosomal position. Scoring 106 may be performed as described in Example 4.

Removing SNPs that overlap with known existing variation 108 may include removing SNPs that demonstrate NMI in the offspring but that overlap with known existing variation. The known existing variation may be within the binding region of an SNP-interrogating probe. In some implementations, the SNP-interrogating probe is used in an SNP assay of the genotyping platform, which may be Illumina. Known variation may include, for example, other SNPs, INDELS (insertions or deletions shorter than the length of the genotyping probe), insertions (larger than the SNP genotyping probe), deletions (larger than the genotyping probe), inversions, and/or mobile element insertions. In some implementations, removing SNPs 108 is performed as described in Example 2 and/or 6.

Identifying conserved regions of the genome 110 may include filtering regions that should be conserved but include a structural variation. Identifying 110 may be performed with CCC (a custom correlation coefficient approach for identification of multi-SNP association patterns in genome-wide SNPs data) as described in Example 3, or with a similar approach.

In screening potentially biologically important SVs 112, the potentially biologically important SV may be any one or more of those SVs that reside in a gene in which less than 5% of normal individuals have a known SV, there is a CCC bloc in the gene (an overlapping CCC region), the frequency of the NMI at one site in the gene is greater than 5% in the diseased population of the study, or there is a run of at least 4 SNPs with NMI in a row.

In some examples, the potentially biologically important SV is an SV that resides in a gene in which less than 5% of normal individuals have a known SV, and there is a CCC bloc in the gene (an overlapping CCC region), and either the frequency of the NMI at one site in the gene is greater than 5% in diseased population of study, or there is a run of at least 4 SNPs with NMI in a row.

In some examples, reference to "normal" individuals may be a reference to data from the 1000 Genome Project. In some examples, reference to a "diseased" population may be reference to individuals with ASD or MS.

In some implementations, screening 112 may be performed as described in Example 4.

In some implementations, screening 112 may help identify NMI that are frequent and/or likely to explain many disease cases. In some implementations, screening 112 may help identify NMI that are large and have a large effect in a few individuals.

Verifying NMI of high frequency 114 may include validating NMI results with a frequency greater than 5%. The validation may include assessing biological and/or functional significance.

Verifying NMI of high frequency 114 in a population may help to determine if NMI of high frequency are true SV. In some implementations, large SV may be readily verified because the probability of having consecutive runs of NMI can be calculated and a significance test performed. In some implementations, NMI of high frequency but a single SNP could be filtered out, such as in screening 112 and/or as described in Example 6. These NMI may be false positives due to normal SV in the human population and/or as a result of poor efficiency of the genotyping platform. Verifying NMI of high frequency 114 may be performed as described in Example 7.

In one embodiment, and with reference to Figure 1B, a method 120 of identifying a genetic structural variation and treating a disease or disorder related to the structural variation is disclosed. The method 120 may include assembling SNP data 122; analyzing for non-Mendelian inheritance (NMI) 124; scoring each NMI 126; removing SNPs that overlap with known existing variation 128; identifying conserved regions of the genome 130; screening for potentially biologically important SV 132; verifying NMI of high frequency 134; and treating a patient 136.

Each of assembling SNP data 122; analyzing for non-Mendelian inheritance (NMI) 124; scoring each NMI 126; removing SNPs that overlap with known existing variation 128; identifying conserved regions of the genome 130; screening for potentially biologically important SV 132; verifying NMI of high frequency 134; and treating a patient 136 may be performed in any order, including concurrently.

Each of assembling SNP data 122; analyzing for non-Mendelian inheritance (NMI) 124; scoring each NMI 126; removing SNPs that overlap with known existing variation 128; identifying conserved regions of the genome 130; screening for potentially biologically important SV 132; and verifying NMI of high frequency 134 may be performed as described above for assembling SNP data 102; analyzing for non-Mendelian inheritance (NMI) 104; scoring each NMI 106; removing SNPs that overlap with known existing variation 108; identifying conserved regions of the genome 110; screening for potentially biologically important SV 112; and verifying NMI of high frequency 114, respectively.

Treating a patient 136 may include treating a patient who has been identified, diagnosed, or confirmed as having a disease or disorder following one or more of the steps 122, 124, 126, 128, 130, 132, 134 of the method 120. In some implementations, the patient has (genetically and/or symptomatically) an autism spectrum disorder. In some implementation, the patient has (genetically and/or symptomatically) multiple sclerosis. In some implementations, the patient has (genetically and/or symptomatically) hereditary hemochromatosis.

In some implementations, the patient is treated with a known intervention, such as a pharmaceutical or non-pharmaceutical approach. Examples of pharmaceutical interventions include small molecules and biologics. Examples of non-pharmaceutical interventions include reducing stimuli (such as reducing noise for a noise-sensitive autistic patient) or physical therapy (such as leg strengthening exercises for a gait-impaired MS patient).

In some implementations, the patient is treated directly or indirectly with a gene editing technology. One example of a gene editing technology is CRISPR. In some implementations, sequence is removed back to the SNPs on either side of the CGV that demonstrate normal Mendelian inheritance. The homologous chromosomal sequence may serve as a guide for with what the SV-altered sequence should be replaced. In some implementations, somatic cells but not germline cells may be altered, which may limit the effect of the editing to the patient and not affect any future offspring.

In some implementations, the patient is treated with CAR T cells. Methods of treating patients with CAR T cells may follow, for example, the FDA-approved gene therapy methods for tisagenlecleucel (Kymriah^{®}, Novartis, Basel, Switzerland) and/or for axicabtagene ciloleucel (Yescarta^{®}, Gilead, Los Angeles, CA). CAR T cells have been approved for treatment of non-Hodgkin's lymphoma and/or for acute lymphoblastic leukemia, and may be employed to treat other diseases or disorder (Boyiadzis et al 2018; Jain et al 2018). In one example, CAR T cells for the treatment of MS target T cells. In one example, CAR T cells for the treatment of ASD target cells involved in the immune response, such as T cells or cells that secrete inflammatory cytokines such as IL-6 or IL-1β. In one example, CAR T cells for the treatment of hereditary hemochromatosis target macrophages.

The presently disclosed methods may be used to identify diagnostic markers, such as networks of genes, for a disease or disorder of interest. The disease or disorder may be any one that has a genetic component. Examples disclosed herein include multiple sclerosis (MS) and autism spectrum disorder (ADS), but the methods are not limited to those diseases and disorders.

### Diagnostic Markers for Autism Spectrum Disorder

The presently disclosed methods identified two different diagnostic networks of genes for ASD. The first is a 14-SNP network that is found in both the parents and the children of families with ASD. As described in more detail in Example 10, a male-only analysis was performed with ~1,000,000 SNP-genotype data from 380 families and controls of the same sex and ethnicity from the 1000 Genome Project. This uncovered a 14-SNP network in 100% of the case families (Table 1, Group I). In order to include females and maximize sample size, genotypes from a second published study were added and the analysis was repeated, which identified the same 14-SNP network plus six other loci (Table 1, Group II).

Given the imputation errors in the 1000 Genome reference, which are described in more detail in Example 13, the allele frequency estimates from those data are likely erroneous but the true frequencies could be determined using newer long-read next-generation sequencing technology. This network is fixed in parents who have children with ASD. In some implementations, the results of the methods disclosed herein may be used as a family planning tool to determine the risk of having a child with ASD. The network also points to possible mechanistic factors that initiate ASD and this assemblage of disrupted genes may cause and have been caused by CGV.

The second diagnostic network of genes for ASD was identified with a combination of NMI (see Example 1) and CCC (see Example 3) to reveal cryptic structural variation in genes that harbor conserved elements in individuals with ASD.

Although demonstrated with ASD, the combination of NMI and CCC may be applied to any disorder or disease that has a genetic component. In some implementations, this method may be used to identify any type of SV as small as a few base pairs and as large as several hundred thousand base pairs.

In contrast, known methods rely on up to nine computational approaches to map short-read technology to a reference (that may contain imputation errors) and then call variants from that mapped reference. In known methods, different approaches are needed to call different types of SV (e.g. deletions vs. inversions) and each layer of statistical inference introduces further bias. Current array-based technology only identifies known SV of relatively large size and of certain types.

### Mechanism of CGV Generation

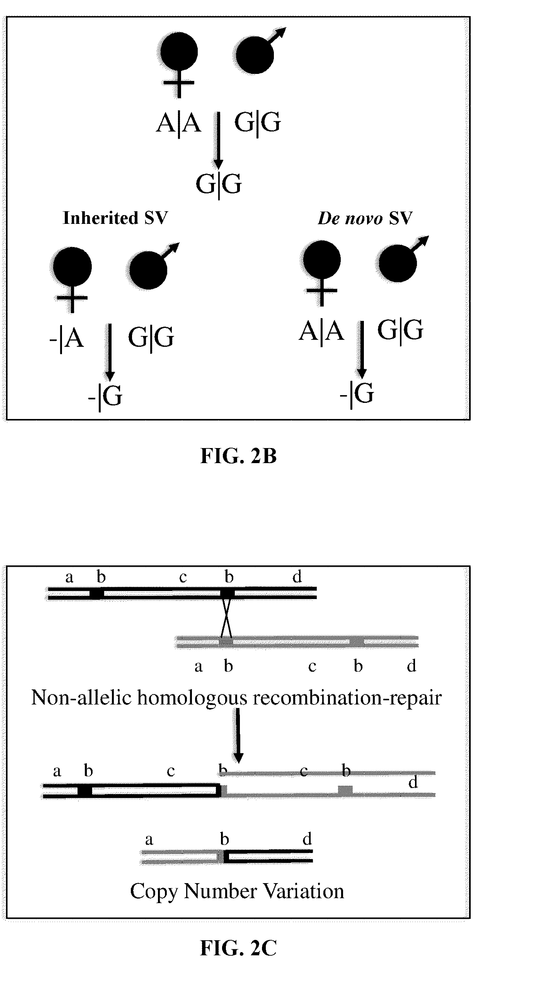

Several pieces of evidence suggest that the 14-SNP network underlies the generation of CGV and was itself caused by previous CGV events via the mechanism presented in (Figure 2C). First, all 14 loci harbor either a simple repeat unit or a Long Interspersed Repeat Element (LINE) near or overlapping the SNP. LINEs are retrotransposon copies that comprise roughly 17% of the human genome and are known to cause CNV. The majority of the LINEs in the 14-SNP network are Type 2, which are older than Type 1 from an evolutionary perspective and tend not to be actively expressed. The MKL2 gene overlaps a Self-chain alignment-a low copy repeat in the human genome that has been demonstrated to generate SVs in general and two genes implicated in ASD. Seven of the 14 SNPs are associated with a simple repeat and in 6 of the 7 cases, the repeats are found to be rare. However, the Polymerase ε (POLE) gene harbors 22 repeats of 64 nucleotides, each of which is delineated by a CpG group. CpGs mark potential methylation sites in the human genome. In this case, the SNP results in the gain of a CpG site and an mRNA folding prediction reveals a highly structured "wheel" that is significantly altered by the mutation and its adjacent, linked polymorphism. This could represent a form of intron delay, which can occur in developmental genes to ensure that their expression and pattern are timed correctly.

In addition to harboring potentially SV-generating repeat units, two of the genes in the 14-SNP network function in processes that can cause CGV when they do not function optimally. POLE and Helicase Lymphoid Specific (HELLS) are components of the meiotic recombination and repair pathways. HELLS is also responsible for de-methylation and re-methylation of CpG sites in the genome during gametogenesis and embryogenesis, the silencing of transposons, and double-strand DNA break and repair.

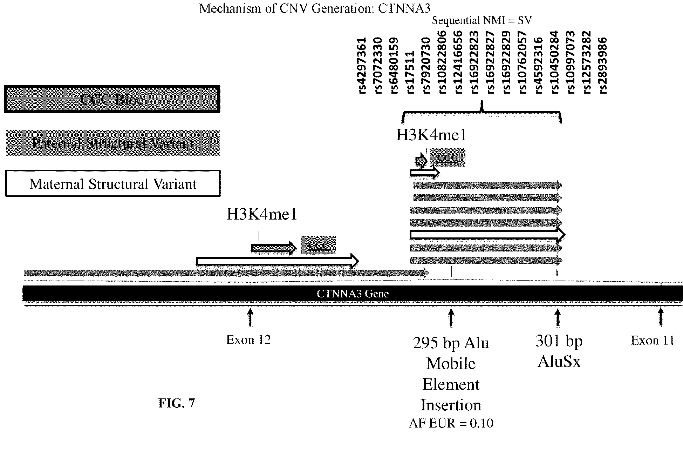

The presently disclosed NMI analysis may identify large deletions by cataloging runs of SV SNPs in each individual, may provide a means to diagnose the full genome of a single person with a simple and cheap method, and may offer further insight into the generation of SVs. As shown in Figure 7, CTNNA3 provides an example. Deletions of exon 11 in CTNNA3 have been reported in individuals with ASD and the presently disclosed NMI analysis identified 10 individuals with a deletion in this region (7 paternal and 3 maternal, Figure 7). The 3' end of the deletion is defined in eight cases by an AluSx element and there is a known mobile insertion element near the 5' end that is present in about 10% of the European population in the 1000 Genome. These two repetitive elements provide a mechanism to expand the SV via non-homologous recombination-repair. In addition, the two CCC blocks identified in this region each harbor an H3K4me1 methylation site that is lost upon disruption of these genomic regions. H3K4me1 is correlated with enhancer elements that may control gene expression. The presently disclosed methods may identify 10 individuals with specific SV and biological relevance. Known methods would miss these genomic alterations because they represent only 1% of the total study population and therefore there is not enough statistical power at the group level to find them.

### Functional Implications of the 14-SNP Loci: Early Development

Three of the loci from the 14-locus network (EPHA8, KCNJ6, and DCX) are involved with neurodevelopment (Table 1). Detailed information is lacking for the MKL2 gene but it likely participates in neurodevelopment because it is a component of the Serum Response Factor, which determines the polarity of the mesoderm and subsequent formation of the neural crest and development of the nervous system. Mutations in ESX1 (which regulates placental development and fetal growth) and DCX (which binds microtubules and is important for the development of the neocortex) could potentially explain the male-bias of ASD because they are both found on the X chromosome.

### Functional Implications of the 14-SNP Loci: Immune Response

Three genes in the 14-locus network implicate the immune response in the development of ASD. Altered immune function has been thoroughly documented in children with ASD including neuro-inflammation, a polarized T-cell response, and microglial activation. In addition to its epigenetic role, the HELLS gene has been shown to be involved in the immune response because it is necessary for the normal proliferation of T-cells. The IL1R2L/IL1R1 genes and their downstream effector IRAK3 also participate in neuro-inflammation, the polarization of T-cells (notably Th1/Th17 skewing and cytokine dysregulation), and the activation of microglia. The IL1R2L pathway can influence the secretion of inflammatory cytokines, such as IL-6 and IL-1β, and the polarization of T-cells in response to infection.

The CHST2 gene encodes for a sulfotransferase that is important for the production of the Lewis X capping group on L-selectin ligands; this capping group is required for optimal L-selectin-mediated lymphocyte homing. Autoimmune diseases occur at higher frequency in parents of children with ASD, circulating levels of IL-6 and IL-1β are elevated in individuals with ASD, and maternal infection during the first trimester of pregnancy is a strong risk factor for having a child with the disorder. Taken together, these associations further implicate immune dysfunction as a major factor in autistic phenotypes.

Particular embodiments of the invention include:
1. A method of identifying at least one structural variation in a genome, the method comprising:
   assembling single nucleotide polymorphism (SNP) data from parents and their offspring;
   analyzing the SNP data for at least one non-Mendelian inheritance pattern (NMI), wherein the NMI is a potential structural variation;
   scoring the NMI to identify large structural variations from sequential SNPs that demonstrate NMI in the offspring;
   removing SNPs that demonstrate NMI in the offspring but that overlap with known existing variation;
   identifying conserved regions of the genome to filter regions that should be conserved but include a structural variation; and
   screening for potentially biologically important structural variation.
2. The method of embodiment 1, comprising verifying NMI of high frequency in a population to determine if the NMI of high frequency are structural variations.
3. The method of embodiment 2, comprising removing SNPs of NMI that are structural variations but that overlap with known existing variation.
4. The method of embodiment 2, wherein NMI of high frequency are present at greater than 5% in the population.
5. The method of any one of embodiments 1 to 4, wherein the potentially biologically important structural variation is selected from one or more of a structural variation that resides in a gene in which less than 5% of normal individuals have a known structural variation; there is a custom correlation coefficient bloc in the gene; a frequency of the NMI at one site in the gene is greater than 5% in a diseased population; and there is a run of at least four SNPs with NMI in a row.
6. The method of any one of embodiments 1 to 5, wherein identifying conserved regions of the genome is performed by a custom correlation coefficient (CCC) analysis.
7. The method of any one of embodiments 1 to 6, comprising assigning a probability on having a run of NMI and maintaining SNPs with a run of NMI greater than 4.
8. The method of any one of embodiments 1 to 7, comprising removing NMI attributable to high levels of masked repetitive elements.
9. The method of any of embodiments 1 to 8, comprising identifying pinpoint locations of the structural variations and identifying pinpoint locations of conserved blocs of genetic information.
10. The method of embodiment 9, comprising using the locations of the structural variations and the locations of the conserved blocs of genetic information to identify locations of rare structural variations in genes that have conserved blocs of genetic information.
11. A method of identifying a structural variation in a genome of a patient and treating a disease or disorder related to the structural variation, the method comprising:
   assembling single nucleotide polymorphism (SNP) data of the patient and their parents;
   analyzing the SNP data for at least one non-Mendelian inheritance pattern (NMI),
      wherein the NMI is a potential structural variation;
   scoring the NMI to identify large structural variations from sequential SNPs that demonstrate NMI in the patient;
   removing as false positives SNPs that demonstrate NMI in the offspring but that overlap with known existing variation;
   identifying conserved regions of the genome to filter regions that should be conserved but include a structural variation
   screening for structural variation related to the disease or disorder; and
   treating the patient identified as having the structural variation related to the disease or disorder.
12. The method of embodiment 11, comprising verifying NMI of high frequency in a population to determine if the NMI of high frequency are structural variations.
13. The method of embodiment 12, comprising removing SNPs of NMI that are structural variations but that overlap with known existing variation.
14. The method of embodiment 12, wherein NMI of high frequency are present at greater than 5% in the population.
15. The method of any one of embodiments 11 to 14, wherein the structural variation related to the disease or disorder is selected from one or more of a structural variation that resides in a gene in which less than 5% of normal individuals have a known structural variation; there is a custom correlation coefficient bloc in the gene; a frequency of the NMI at one site in the gene is greater than 5% in a diseased population; and there is a run of at least four SNPs with NMI in a row.
16. The method of any one of embodiments 11 to 15, wherein identifying conserved regions of the genome is performed by a custom correlation coefficient (CCC) analysis.
17. The method of any one of embodiments 11 to 16, comprising filtering out of structural variations that are not specific to the disease or disorder.
18. The method of any one of embodiments 11 to 17, wherein the treating includes a gene editing technology.
19. The method of embodiment 18, wherein the gene editing technology includes CRISPR.
20. The method of embodiment 18, wherein the treating includes administration of CAR T cells.

### EXAMPLES

The following examples illustrate various aspects of the disclosure and should not be considered limiting.

### Example 1 - Samples and Sources

Parent-child SNP genotypes from families (case (study subjects) and their parents) were obtained from the database of Genotypes and Phenotypes (dbGaP). The first consists of 1,177 individuals that represent 381 families genotyped at 1,048,847 nuclear SNP loci in a study at the University of Miami. Data from the second study was produced by the Autism Genomic Project Consortium (AGPC), and consists of 4,168 individuals representing 1,385 families genotyped at 1,072,657 nuclear loci. The genotypes for the Multiple Sclerosis study described below were obtained from dbGaP (phs000139.v1.p1 and phs000171.v1.p1). The control data for the CCC BlocBuster analysis described below (Example 3) were the 503 individuals of European descent from the 1000 Genomes Project. Data were handled in accordance with the rules established by the National Institutes of Health and Washington State University to preserve confidentiality of patient information.

HapMap data were used as an additional control and to test for possible imputation errors in the 1000 Genome dataset. The final data were derived by merging genotype calls from two different platforms, the Illumina Human 1M and Affymetrix SNP 6.0, after evaluating Hardy-Weinberg equilibrium (p > 0.000001), levels of missing values (< 0.05), and Mendelian errors (< 3); as well as checking for concordance between the 249,889 overlapping SNPs.

### Example 2 - Data Quality Control and Formatting

Potentially erroneous calls were removed from the samples of Example 1 by excluding all SNPs with a quality score of less than 0.75. One family was removed from the Miami data set and two from AGPC due to poor data quality and 248 families were removed from the AGPC data set because they did not have a quality score. After an initial analysis with CCC (see Example 3) several categories of false positives were identified that in many cases were "fixed" for one allele: (1) those that were paralogs such that the variant was a paralogous sequence variant (PSV) and not an SNP, (2) SNPs for which there were conflicting allele frequencies in public databases, and (3) X-linked SNPs that reflected the sex bias in the data sets (there are significantly more males with ASD than females). Many SNPs of the third category were in the gene protocadherin 11X, for which there is a highly conserved homolog (protocadherin 11Y) on the Y chromosome. These genes are located on the non-recombining portions of the sex chromosomes, but the Illumina probes in many cases matched the SNP in both loci and accordingly were analogous to a PSV. After the identification and removal of these false positives, the CCC analysis was performed once more to ensure that a potential network was not a spurious result due to the PSV.

For the protocadherin locus, all SNPs that presented as "heterozygous" were identified and all markers for which the Illumina probe matched both the X and Y variants were removed. To identify potential PSVs in other genes, all SNP loci that were fixed in the ASD data set and were at an allele frequency of 0.8 or less in the 1000 Genomes European data set were extracted. From the NCBI dbSNP map viewer, a 2kb of sequence on either side of each of these 339 loci was extracted and reads were mapped onto them from the FASTQ files downloaded from the NCBI Sequence Read Archive (SRA) for one individual from the 1000 Genome Project. From the assembled reads, 112 of the loci appeared to be paralogs (there were multiple SNPs surrounding the targeted SNP that would not have allowed the binding of the Illumina probe). The Illumina probe did not match the SNP sequence provided by dbSNP in 116 of the remaining loci, eight were listed as "SUSPECTED" in dbSNP, and the final 103 appeared to be true SNPs. The potential false positives were removed from all of our datasets as were any C/G and A/T SNPs because it could not be determined which strand was scored in the data from the dbGaP studies.

### Example 3 - BlocBuster (CCC)

The CCC (custom correlation coefficient) algorithm was developed as a component of the program BlocBuster. This algorithm identifies evolutionary conserved blocs of a genome. The blocs may be regulatory regions that control the expression or splicing of a given gene. CCC has not previously been combined with NMI analysis. Compared to known methods of genetic analysis, the presently disclosed methods, including the combination of CCC and NMI analysis, helps permit accurate identification of CGV.

Two data sets generated from Example 1 were used for the CCC analyses: the first data set included all of the males' genotypes from the data produced by the University of Miami study and the second used the University of Miami individuals and added individuals from a second study from the Autism Genome Project Consortium to account for the sex bias in ASD. In the first data set, only males were analyzed for the first run (N=336) and for controls were of the male individuals of European descent (N=240) from the 1000 Genome Project (only genotypes from children and not parents were used for the CCC analysis). The results from that analysis are listed as "Group I" in Table 1. To analyze the female genotypes, sample size was increased by combining the Miami and AGPC datasets for a total of 471 cases (234 females and 237 males). The same number individuals of each sex were randomly chosen from the European individuals in the 1000 Genome Project as controls. This is "Group II" listed in Table 1.

The CCC program is computationally intensive and can take many computer CPU hours to run. However, the scalability is logarithmic and therefore, reducing the number of SNPs by half decreases processing time by an order of magnitude. This also has the desirable property of removing CCC correlations that are due to physical linkage on a chromosome. To do this, for each of the CCC analyses (Miami only and the AGPC&Miami), the data were divided into two data subsets to speed processing and to reduce effects of linkage disequilibrium: first, the data was sorted by chromosome and position and then every second SNP was taken for the first data set and the remaining comprised the second. Based on smaller sample runs, a single file of 916,847 SNPs was estimated to take approximately 29 computer days to run, but two files of half the size each take 2.9 days. Typical simulations with permuted data to identify a correlation cutoff for CCC was not possible given the size of the data files. Instead, a cutoff of 0.75 was chosen to be conservative and the highest 20% of the edges were kept so that the number of edges scaled to the two different data sets (380 families versus 1,137). In this case the simulations were not relevant because a single network was identified and it was fixed in families with ASD. Using a range of correlation cutoffs (0.70-0.80) gave the same result.

### Example 4 - Non-Mendelian Inheritance (NMI)

The program PLINK was used to identify SNP loci that did not conform to Mendelian inheritance and therefore represent CGV (see Example 9). Programs similar to PLINK may also be used. In most observed cases of NMI, the expectation was that the child should be heterozygous but instead displayed homozygosity at a site (e.g. parental genotypes were "A/A" and "G/G" and offspring was "G/G"). The "mendel" function in PLINK outputs codes that can be directly translated into paternal or maternal errors. In addition, we scored scenarios where genotypes were one of the following: child = "A/A", father = "A/A", and mother = "-/-" as maternal CGV (and paternal where, similarly, the genotype is missing for the father but present in the mother). All genotypes that were flagged by PLINK in the ASD children were labeled as an NMI with a score of 1.

Although PLINK was used for part of the analysis, the program has limits that have not been previously identified. Specifically, the state of the art has been that a missing genotype is a failure of the genotyping platform. It has not been previously recognized that a missing genotype is a failure of the platform to genotype because the target piece of DNA is not present in the evaluated individual or the DNA has been altered by structural variation. Further analysis was performed that is unique to the presently disclosed methods. Specifically, in some cases, multiple adjacent SNPs in one or more individuals in a trio gave this null signal ("-/-") but one or two family members demonstrated normal, homozygous genotypes at all the loci. Genotypes of "-/-" in the ASD children were labeled as an NMI with a score of 1, along with the PLINK-flagged genotypes described above. These data were used to calculate the frequency of NMI across the population.

Compared to known methods, the presently disclosed methods include assigning a probability on having a run of NMI. The inclusion of NMI run probability may help to identify genomic "tears." Specifically, to identify large CGV (runs of CGV in each individual), a running sum on position-sorted NMI with a window size of 5 was calculated. Based on the probability of identifying runs of NMI by chance, a cut off of 4 or more was used as significant (1 x 10⁻⁶). All loci were kept in which at least one individual had a run of NMI >4. These were then filtered further for gene-level overlapping CCC blocks and existing known SV.

### Example 5 - Identification of Structural Variation (SV) in the 1000 Genomes

In this Example, publicly available data was used to filter out CGV that was not specific to ASD or MS. This filtering may help permit the detection of biologically relevant CGV (which can be employed in one or more of Examples 11, 12, and 14, below). To extract the structural variation from the latest Build of the Human Reference Genome (hg38), all tracks through the Reference Data Manager portal in CLC Genomics Workbench (version 12) were uploaded, structural variant files (ftp://ftp.ebi.ac.uk/pub/databases/dgva/estd219_1000_Genomes_Consortium_Phase_3_Integrate d_SV/) were uploaded, and for each SV track annotations were merged with a BED file delineating each position of our 63,868 NMI SNPs. For each SV type (gain, loss, inversion, and mobile element insertion), all SV identification numbers were extracted that overlapped with these 63,868 regions as potential normally segregating variation in the human population. VCFTools was then used to extract the genotypes for all SV IDs from the 1000 Genome population. The frequency of each SV in each of the five populations (African, American, East Asian, European and Southeast Asian) was calculated. In some cases, multiple SVs overlapped a given SNP; for these cases, the SV with the highest frequency for that SNP location was identified. To be conservative, any NMI SNP was removed from further analysis that had a frequency of greater than 5% in the EUR population for all SV types (the max frequency of all types was used for this calculation). This approach was conservative in assuming that any NMI identified was a normally segregating variant and unlikely to be involved in causing ASD.

Compared to known methods, the presently disclosed methods included additional removal of non-Mendelian hits that could be due to high levels of repetitive elements that are "masked" from downstream analyses, which is a common feature in genomes. Specifically, to determine if a repeat element (such as Short Interspersed Nuclear Elements-SINES-or Long Interspersed Nuclear Elements-LINES) overlapped our NMI and CCC SNPs from Examples 1 and 3 , the RepeatMasker track in BED format from UCSC Genome Table Browser (https://genome.ucsc.edu/cgi-bin/hgTables) was uploaded to CLC Genomics. Annotations were overlapped with the SNPs with a range of 50bp on either side of the SNP of interest that could potentially interfere with the binding of the Illumina probe. The same analysis was performed for all SNPs on the Illumina array (1,012,113) to generate an expected frequency for our NMI and CCC data sets. Counts were binned into categories of different transposable elements: ALR/Alpha, Alu (SINES), HERV, LINE1, LINE2, MAM, MIR, THE1, Charlie, HAL, LINE3, LINE4, LTR, MER, MIR, MLTF, and Tigger. A Chi-Square test was done using the frequency from the full Illumina array to generate the expected number of elements in each category for each group (all NMI, NMI with runs greater than 4, and CCC SNPs). A Bonferroni correction (p < 0.002) was used to account for multiple tests.

The expectation is that there will be no enrichment for any of the foregoing classes of repetitive elements in genomics regions with SV. If there are enrichments for certain types of repetitive elements in the disease data compared to the data from normal individuals, based on expected frequency (generated from the frequency of each element genome-wide), this may indicate biological relevance. For example, the transposon may be a part of the SV process for a given disease. In the case of Autism, there is an enrichment for active (L1 - LINE1) transposable elements and a decrease in the expected number of inactive (L2) elements. L1 transposons are correlated with SV in Autism and may be the underlying cause of the disorder.

### Example 6 - Removal of INDELS and Null Alleles

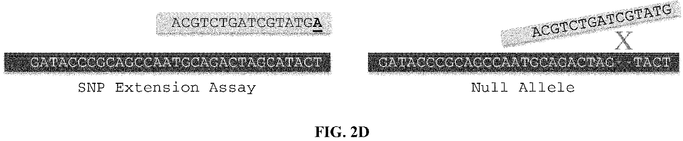

In this Example, CGV that was not specific to ASD or MS was filtered out. Specifically, NMI can also be caused by smaller cryptic variation such as INDELS or SNPs that are located under the 50bp probe used in the Illumina bead chip assay (null alleles; see Figure 2D). These are likely normal genetic variation in the human population yet are detected as false positives. To eliminate nulls caused by INDELS or other SNPs under the genotyping probe, the latest dbSNP 150 database was used to generate an NMI track that included sequence +/- 50bp from the NMI SNP, and identified any INDELS with a MAF >5% from the 11,783,130 INDELS in the dbSNP 150 database. The same was done for null alleles by identifying SNPs within 50bp of the NMI SNP that had a frequency of greater than 5% in the CEU population (those of European ancestry that match the ethnicity of the ASD population) from the 1000 Genomes. These were removed from further analyses as was the case for Examples 2 and 5 to remove false positives.

### Example 7 - Linkage Disequilibrium and Heterozygosity Analysis

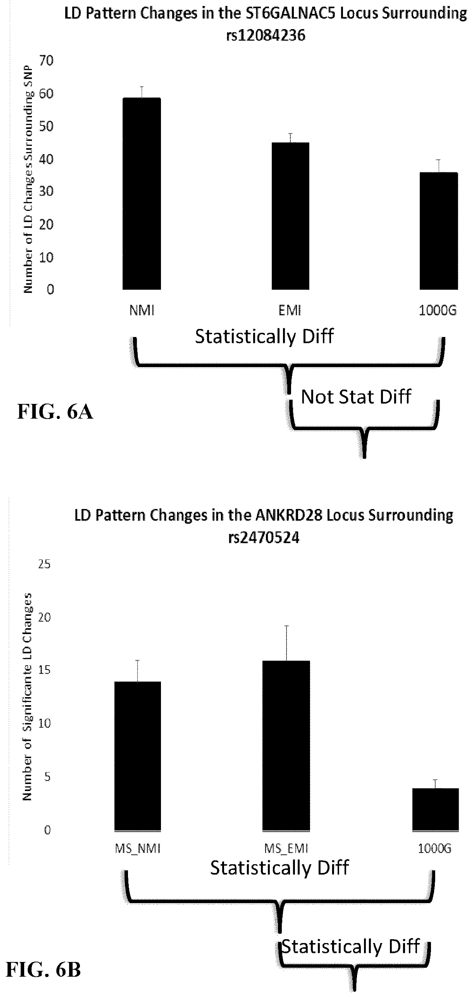

This Example was designed to verify that NMI of high frequency are true SV. For large SV, this may be easily done because the probability of having consecutive runs of NMI can easily be calculated and a significance test performed. But for NMI of high frequency but a single SNP, this could be a false positive due to normal SV in the human population, which were filtered out in Examples 2, 5, and 6. It could also be a false positive SV due to poor efficiency of the genotyping platform. To test for that, the connections were measured between all of the SNPs surrounding the single SNP with NMI and high frequency. For each of these SNPs, genotypes from the 10 SNPs upstream and downstream from the site were extracted from the cases and the 1000 Genome Project. The LD function in the "Genetics" package in R was then used to calculate the 1,805 pairwise LD values for the 20 SNPs surrounding the NMI site by randomly sampling 50 individuals, 10 times. This was done for 3 groups: the 1000 Genome Project (external control), the individuals that demonstrated normal expected Mendelian inheritance patterns at the site (internal control; "EMI" in Figures 6A-6C), and the individuals that demonstrated non-Mendelian inheritance at the site ("NMI" in Figures 6A-6C). The number of pair-wise LD values that changed from significant to non-significant or non-significant to significant for each of the 10 re-samplings for the three groups was enumerated. From these data, the mean and standard error for each group was calculated and statistical significance (p < 0.05) was determined with a student's t-test. For determining neighborhood disruption, the *overall* number of changes in LD among groups may not be as valuable as the number of *site-*changes (i.e., whether or not the *pattern* of LD changes).

There are three expectations from this analysis: (1) the pattern of connected SNPs within this region are the same for all three groups and the NMI is therefore due to genotyping error; (2) the NMI is greater than both EMI and 1000G and individuals have been efficiently categorized with the true SV in the NMI category; and (3) both NMI and EMI are greater than 1000G and therefore this is a true SV, but there are individuals in the EMI group we have not successfully identified. This can happen in the case where the genotypes are Mother = A/A, Father = -/A, and child = -/A; there is no power to detect the NMI with the test in Example 4.

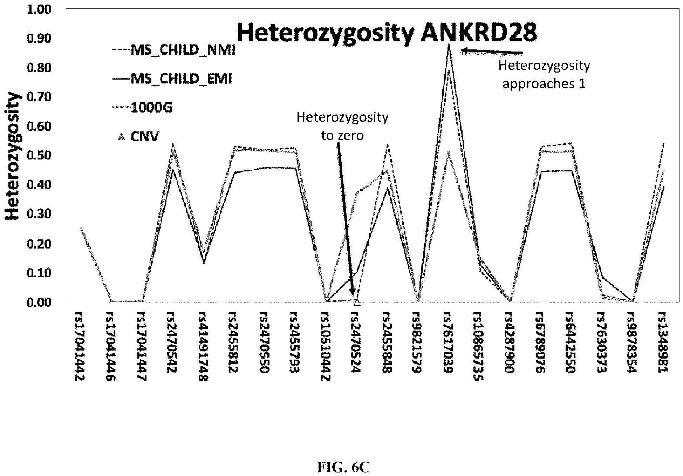

In this third case where EMI appear to have individuals with SV that have not been efficiently identified, the heterozygosity surrounding the NMI site in all of the individuals (children and parents in EMI and NMI plus the 1000Genome control) can be plotted to further verify that this is an SV. The heterozygosity should drop in all family members compared to the 1000 Genome in both EMI and NMI categories. As shown in Figure 6C, the NMI heterozygosity drops to zero at the NMI focus SNP (rs2470524), and for the EMI group, it approaches zero but a few individuals that are heterozygous remain. These results demonstrate that this is a true SV, and is at high frequency in the MS population. Additionally, the heterozygosity plot identifies a second SNP to the right (rs7617039) where the heterozygosity approaches 90% in the MS population. These two data points indicate that an SV in this section of the ANKRD28 gene exists in most individuals with MS and is likely the cause of the disease. Other SV mentioned herein contribute to the severity.

The use of "neighborhood LD" to determine if an NMI SNP is simply a genotyping error or if there is true SV in the region is shown in Figure 6. In each of Figure 6A and 6B, the SV is verified because there are significantly more LD changes compared to the 1000 Genome control when the focus NMI is removed from the analysis. However, in Figure 6B it appears that the EMI group still contain individuals with NMI: there is an SV, but the NMI test does not have power here due to low heterozygosity (parents and kids are homozygous for one allele type). This was verified with a heterozygosity plot (Figure 6C). SV was verified with the drop in homozygosity in both groups, and an adjacent site was identified in which heterozygosity approaches 1. This further verifies the SV and suggests copy number variation exists in nearly all individuals with MS at this location.

### Example 8 - Functional Analysis and Disease Association

Enrichment for biological, cellular, and molecular function were carried out with the Gene Ontology database (http://geneontology.org/). Standard False Discovery Rate (FDR) cut offs were used. Functional analyses for specific genes were taken from GeneCard Human Gene Database. Google Scholar^{™} and the Online Mendelian Inheritance in Man were used to search for association with disease. All gene sequences were annotated with CLC Genomics Workbench (version 12 Hilden, Germany). RNA folding predictions were performed with the web-based software at the University of Vienna.

### Example 9 - Initial Discovery of Non-Mendelian Inheritance (NMI) and Structural Variation (SV)

The present state of the art does not appreciate that non-Mendelian inheritance (as in Example 4) can be used to identify CGV and non-Mendelian inheritance is accordingly not used to identify CGV. Compared to known methods, the presently disclosed methods employ non-Mendelian inheritance, add filters to remove "normal" structural variation, and add the CCC filter (described in Example 3) to identify regions that should be conserved (unchanged across evolutionary time) but are disrupted and specific to a disorder of interest.

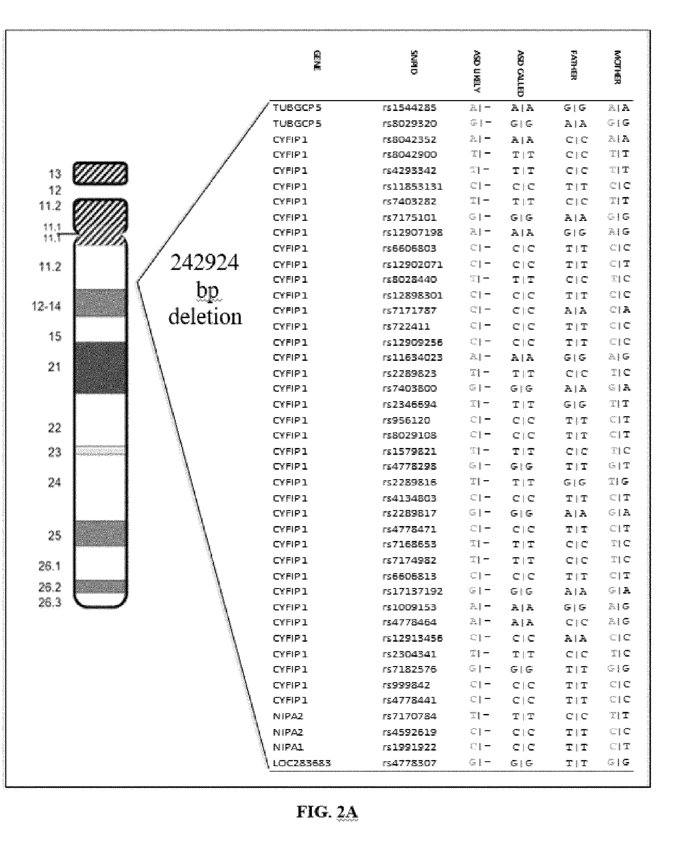

In a previous analysis, genotypes were phased in the individuals from these ASD families leveraging the parental information and many loci indicated non-Mendelian inheritance patterns. Upon further inspection, it was noticed that in most cases the rejected locus consisted of numerous (in some cases more than 100) SNPs that were physically adjacent on a chromosome, and that some loci had previously been reported as chromosomal deletions in neurodevelopmental disorders (Figure 2A). One possible explanation was that the non-Mendelian inherited loci were haploid sections of DNA that were the result of CNV-yielding deletions, which can arise from aberrant recombination-repair processes, and have been heavily implicated with ASD (Figure 2C). In addition, these CGV could also represent null alleles caused by INDELS that disrupt the binding of genotyping probes (Figure 2D). The probability of identifying consecutive NMI can be calculated with a simple binomial distribution because Illumina bead arrays randomize SNP genotyping assays for every sample. Based on the overall NMI rate in both data sets, the probability of obtaining NMI in 38 consecutive draws (Figure 2A) is 8 x 10⁻¹⁰⁶, which is nearly impossible.

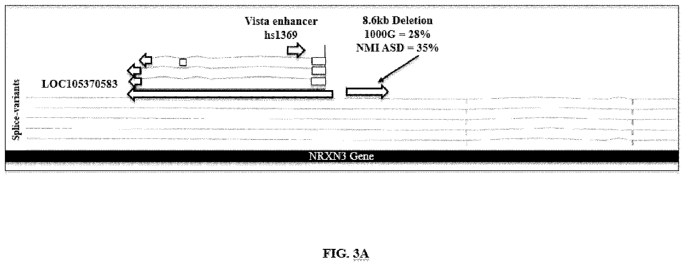

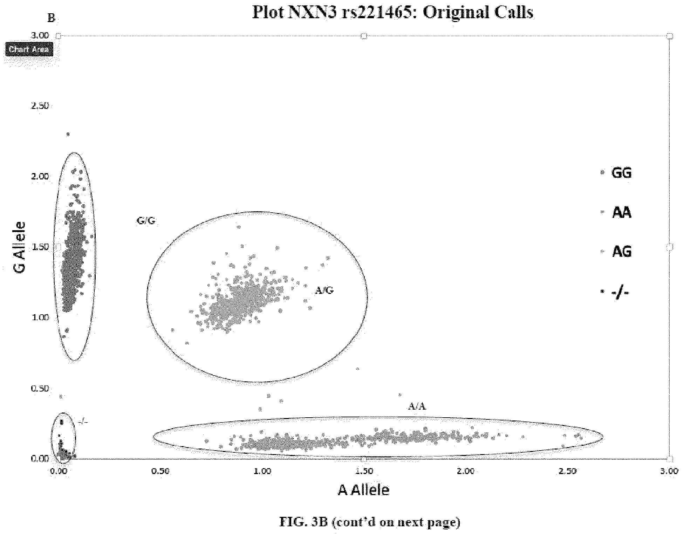

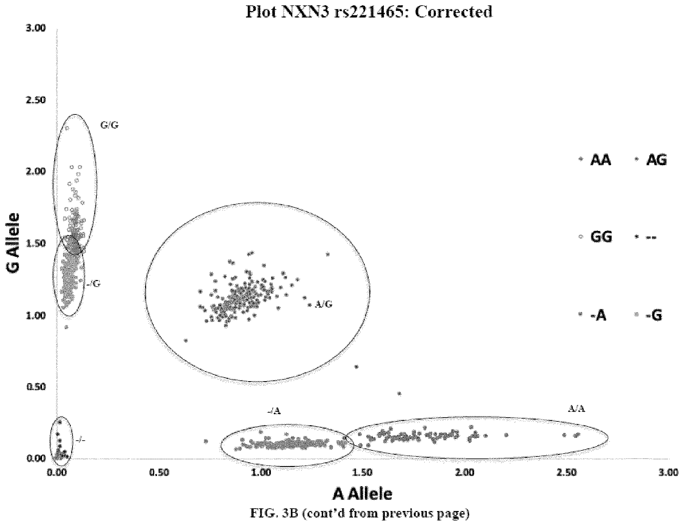

In contrast to the above example, in many cases there was a single NMI found as in Example 4 that was found at high frequency within the population. This was the case with the NRXN3 gene, which functions in the formation of synapses and has been associated with ASD in previous work. Furthermore, the SNP (rs221465) resides at a biologically interesting location in the NRXN3 gene (Figure 3A); it is proximal to an ncRNA near an intron/exon border, a methylation site, and an enhancer that is expressed during neural tube development. However, the new release of the Human Genome (hg 38) identified a deletion at this location with an allele frequency of 0.28, roughly matching our NMI frequency. The raw intensity values were plotted for each allele and parental information was used to re-genotype for the deletion (Figure 3B). The allele frequencies for the deletion indicate normal Mendelian inheritance and conformation to Hardy-Weinberg Expectations (HWE). The results indicate that known structural variation in the human genome (in this case a deletion) can be identified. The results may identify NRXN3 as a false-positive marker for ASD. Compared to known methods, the presently disclosed methods permit the filtering out of CGV that are not specific to the disorder or disease of interest as in Examples 2, 5, and 6.

### Example 10 - BlocBuster Analysis

Because ASD disproportionally affects males, a male-only analysis was performed with ~1,000,000 SNP-genotype data from 380 families and controls of the same sex and ethnicity from the 1000 Genome Project. This uncovered a 14-SNP network in 100% of the case families (Table 1, Group I). In order to include females and maximize sample size, genotypes from a second published study were added and the analysis was repeated, which identified the same 14-SNP network plus six other loci (Table 1, Group II). Two SNPs from Group II (rs7996725 and rs5942883) were removed because the latest version of the human genome (hg38) identified INDELS within the sequence of the Illumina probe with allele frequencies that could explain the differences between the ASD and 1000 Genome data due to probe failure. A third SNP (rs2690904) was not listed in hg38 and was therefore discarded. The remaining 17-SNP network was found in 78% of the case families. More than half (10/17) of the loci have been implicated in ASD in previous work and two others have been associated with developmental delay. Extraction of genotypes from all individuals in both data sets demonstrated that the alleles at many of the SNP loci are fixed in the children with ASD, are inherited from their parents, and are either absent or at low frequency in the 1000 Genomes data from individuals of European ancestry.

All 17 of the SNP loci were within 50 basepairs of a SV that included transposons (LINE and HAL1 elements), simple repeats, INDELS, and Self Chain low copy repeats, which have been shown to induce mutations in MECP1 and NRXN1 (both associated with ASD) (Zhou et al., 2013). With the exception of the POLE gene, the simple repeats and INDELS are reported to be rare in the human genome (< 1%) and are unlikely to explain the fixed frequency found in the ASD data set. These repeats, however, provide a potential mechanism for SV generation through sub-optimal recombination-repair. In addition, 9 of the 17 have an NMI elsewhere in the gene, which provides further support for that mechanism.

### Example 11 - Filtering of Structural Variation and Biological Significance

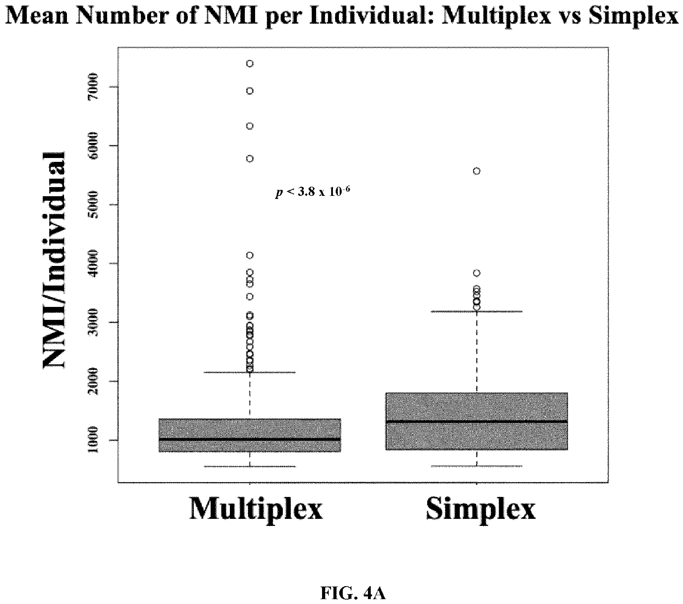

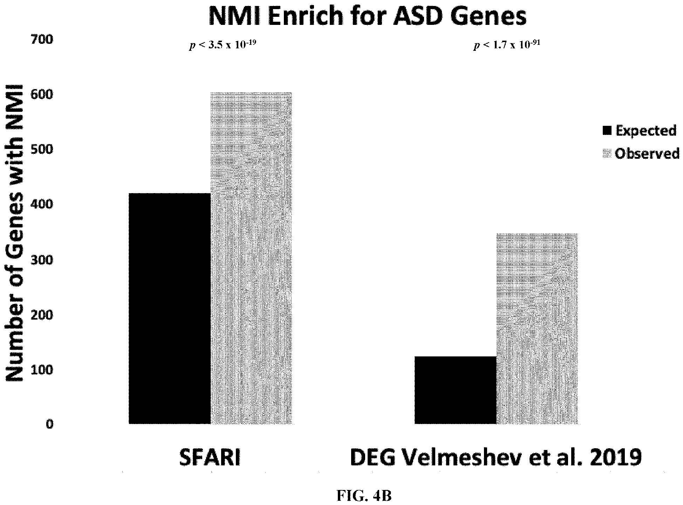

Results generated in the preceding Examples demonstrate that NMI is an accurate means to identify SV based information from known SV in the 1000 Genome data (e.g. NRXN3). In order to determine which loci (if any) were biologically significant with respect to ASD, differences in the proportion of NMI between the phenotypes of Multiplex and Simplex children (families with more than one child with ASD compared to those with a single child) were tested. There is a statistically significantly greater number of NMI loci in the Simplex compared to the Multiplex families (Figure 4A, *p* < 3.8 x 10⁻⁶). This fits with published reports demonstrating that children from Simplex families have more severe forms of ASD than those from Multiplex families. Next, the data was examined for an enrichment of ASD-associated genes in NMI regions of the genome that had been identified. The SFARI database lists 1,053 Autism-causing genes (https://www.sfari.org/resource/sfari-gene/), of which 997 are identified by at least one SNP on the Illumina array used for the two studies here. Of the 17,580 protein coding genes that overlap with the SNPs on the Illumina array, 7,628 (43%) have at least one NMI associated with them. Based on this frequency, one would expect to find an NMI in 433 of the 997 SFARI ASD genes, but instead NMI were identified in 606; a significantly greater number (Figure 4B, p < 7.6 x 10⁻¹⁷).

A recent report used single-cell transcriptomics on post-mortem brain tissue from ASD and matched cases to identified 513 differentially expressed genes (Velmeshev et al., 2019). Most of the SV identified in this study are in non-coding regions and would be more likely to disrupt gene regulation than the amino acid sequence of the protein. Enrichment of large SV (those with a run of NMI >4) in these differentially expressed genes was tested. Of the 513 listed in that study, 475 were represented by the SNPs on the Illumina array used in the two ASD studies here. For this test, 29% of the genes from the Illumina array had an NMI in these categories; all expressed genes were included from the Illumina array that matched the categories of the 475 genes from the single-cell transcriptome study of Velmeshev et al. 2019 (anti-sense, lincRNA, processed pseudogene, processed transcripts, protein coding, transcribed processed pseudogene, transcribed unprocessed pseudogene, unprocessed pseudogene). This is in contrast to the test with the SFARI database, which included only protein-coding genes. The expected number of genes from Velmeshev et al. 2019 (N=123.8) is significantly lower than the observed (N=348, p < 1.7 x 10⁻⁹¹, Figure 4B). The results showed that over 70% of the genes that differ in expression in ASD brain tissue have an NMI from the presently disclosed methods in them. The SV that is identified by these NMI may directly alter gene expression. The presently disclosed methods can also identify the differentially expressed genes that are compensatory to the SV-those that are altered but have no SV.

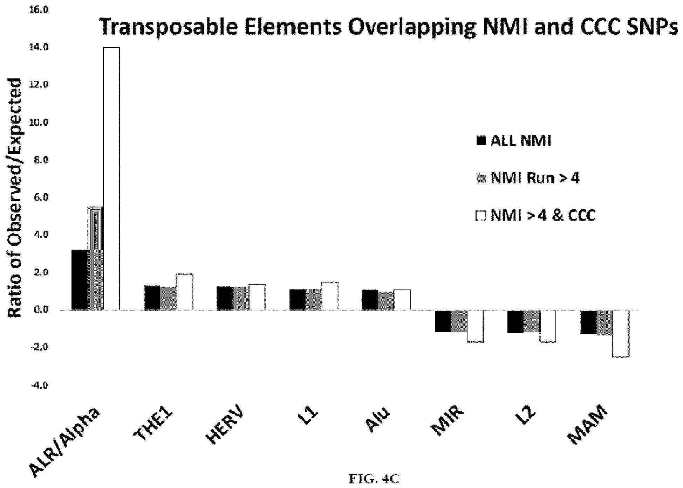

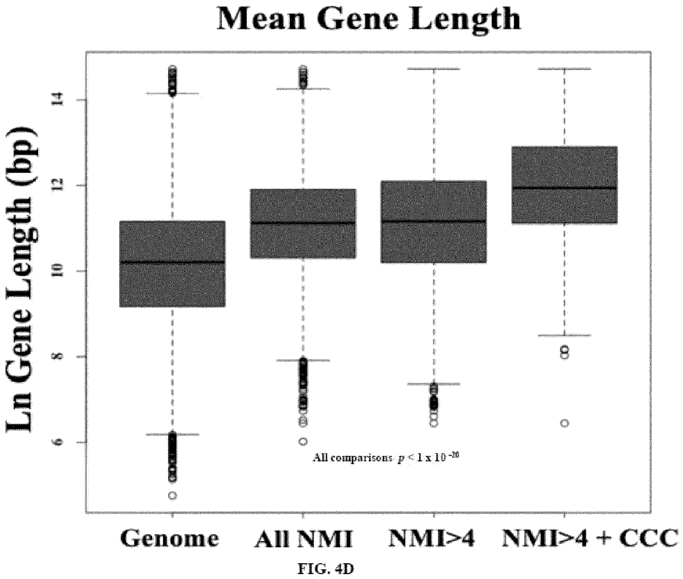

Also shown in Figure 4, specifically Figure 4C, is that the genes in which the SNPs reside overlap a greater number of several transposable element types, notably ALR-alpha and LINE1 elements. They show less than expected overlap with LINE2 and MIR. There is a large increase in NMI in conserved ALR repeats, which are an important component for the attachment of microtubules for meiosis and mitosis. LINE1 include transposons that are active in the human genome whereas LINE2 tend to be inactive and older than LINE1. MIR are highly conserved elements that appear to regulate gene expression levels in the human genome. Figure 4D shows NMI enrich for longer genes. Previous work documented that ASD genes are larger and contain more conserved repeats such as TEs than other genes.

Another means to use NMI to filter biological relevance is to look for functional enrichment of those with the highest frequency as opposed to those that are large (NMI >4) (see Examples 7 and 14). NMI with a frequency of greater than 20% in both the Miami and AGPC ASD data sets (Example 1) were filtered out. A Gene Ontology analysis (as in Example 8) identified a 100-fold enrichment for H3K9 tri-methylation, which is a type of modification to a chromosome that effects gene expression, and is necessary for proper embryonic development and X-chromosome inactivation. In the ASD data, 88% have at least one SV in one of these 11 genes that participate in H3K9 methylation. All of the genes reduce H3K9 methylation and therefore improper function from an SV would cause increased H3K9 methylation, which is what has been reported in many cases of ASD.

### Example 12 - Structural Variation in Differentially Expressed Genes

The CCC algorithm (see Example 3) identifies blocks of SNPs that are evolutionary conserved or possibly function in epistasis. CCC can provide information on where biologically important SV are located. The presently disclosed methods also sought to identify genes with SV that also harbored conserved elements with the assumption that disruption of genetic regions that are meant to be conserved would be biologically meaningful in the context of disease or disorders. Compared to known methods, the presently disclosed methods identify both where SV are located and where conserved blocs of genetic information are located. The combined information can be used to identify locations of rare SV that are in genes that have conserved blocs of information. Such analysis can be performed on an individual basis.

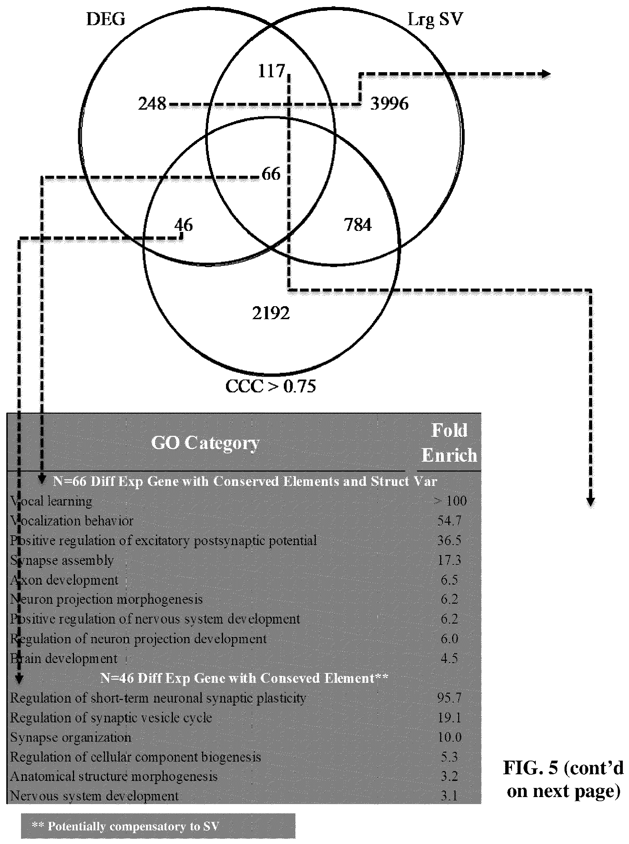

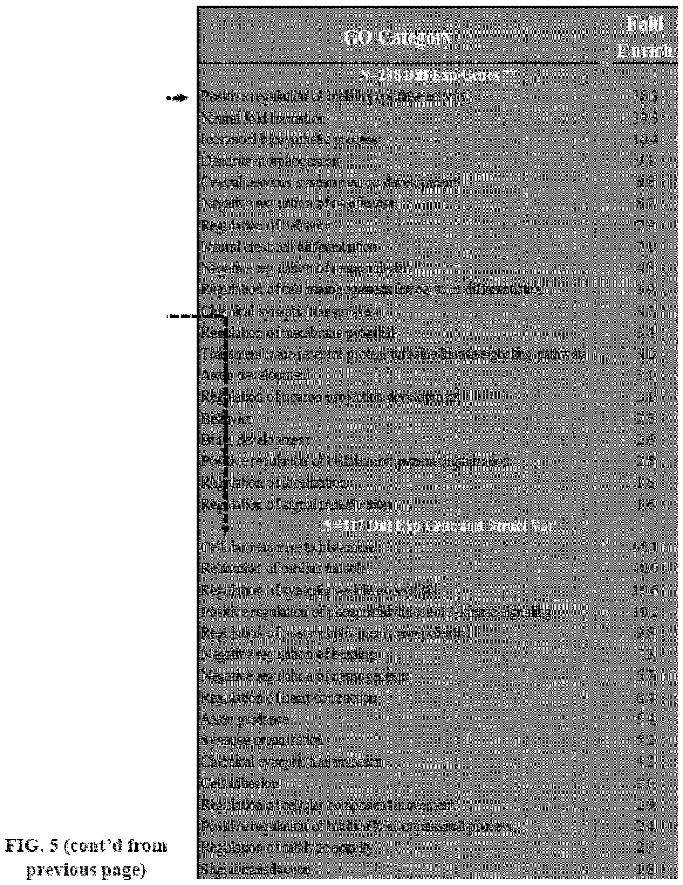

First, the 513 genes from Velmeshev et al. 2019 were filtered for structural variants and blocs of CCC to determine if there was a functional enrichment. Of the 477 genes from that study that were represented by the Illumina array used in the studies here, 66 had both a CCC bloc and a large SV (NMI run >4), 46 had only a CCC bloc, 117 harbored a large SV but no CCC, and the remaining were differentially expressed genes (DEG) that did not have a detectable CCC bloc or a large SV. An analysis with Gene Ontology revealed a 100-fold increase in the category of "Vocal learning" for the 66 genes (FDR < 0.05, Figure 5). Notably, the greatest fold-enrichment for the DEG-only category (N=248) and the DEG with CCC but no SV (N=46) are both involved in neural plasticity. This may be because these DEG are compensatory to those caused by SV. The gene VWA8, which is in the 14-SNP CCC network (Table 1) was found to be downregulated in the single cell transcriptomic data from Velmeshev et al. 2019, supporting the results disclosed herein that it harbors an NMI that effects its expression. This gene was also identified as a strong but not statistically significant locus in the original GWAS (it was referred to as KIAA0564 in that work).

In Figure 5, structural variations were those with NMI loci that appeared in consecutive runs of 4 or more, and with no known structural variant in the 1000 Genome reference greater than 5% in the EUR populations. The differentially expressed genes were those that were discoverable on the Illumina array used in the original ASD studies and on autosomes (NMI were not calculated on the X-chromosome). The Gene Ontology (GO) Category and fold-enrichment were calculated for the four sub-groups of the differentially expressed genes. Those with a double asterisk may be compensatory to the others because there is no known structural variant in these genes that could disrupt gene expression. The top hits for these two categories are both central to neural plasticity, which is what one would expect as a compensatory action.

### Example 13 - Identification of Imputation Errors in the 1000 Genomes Reference Database

Imputation was used extensively in producing the 1000 Genome data because it does not allow for missing genotypes, but where and when it was used was not precisely recorded (http://www.1000genomes.org/). A true deletion in the human genome would be erroneously scored with imputed genotypes in this framework. Data for nine of our 14 SNP loci as well as one of the three additional SNPs shown in Table 1 for Group II were available in archived HapMap genotypic data that was not subject to imputation. Forty-eight percent of the data were missing for the nine loci and approximately 5% at the three additional loci.

Forty-eight percent of the genotypes were not randomly missing from this subset, rather 48% of the *individuals* were missing genotypes at *all* nine loci and 5% of the *individuals* were missing genotypes at *all* three of the additional loci. One possible explanation is that these genomic regions were erroneously imputed in the 1000 Genome database. This suggests that the allele frequencies of the 1000 Genome Reference data likely do not reflect the true values. All known structural variants in the human genome were ruled out as possible causes of probe failures for one of the alleles at these loci. These included known CNV, insertions, deletions, mobile element insertions, inversions, INDELS, and null alleles due to SNPs that occur where the Illumina probe binds. One possibility that explains the results are that there are SVs in these regions that have not been identified and the 1000 Genome was erroneously imputed. Another possibility is that these data reflect true allele frequencies specific to the ASD families. Compared to known methods, the presently disclosed methods are able to identify erroneously imputed regions of the 1000 Genome date.

### Example 14 - Application to Multiple Sclerosis

The method 100 (Figure 1) was performed on a second set of data from 454 families with an individual diagnosed with MS and genotyped at roughly 400,000 SNPs. Eighty seven genes were extracted that had a run of four NMI or more, less than 5% known SV from public data, and harbored at least one CCC network block with a correlation cutoff > 0.75 using Examples 1-11. A Gene Ontology analysis indicated a 100-fold enrichment for genes involved in the 2'-5'-oligoadenylate synthetase pathway. This pathway is involved in the interferon-induced anti-viral pathway and has been implicated in MS.

The frequency-based analysis identified 317 genes that contained an SNP with > 5% NMI frequency across the population, did not have known SV > 5%, and harbored at least one CCC network with a correlation value > 0.75 among SNPs. The top hits explain every case of MS represented by two biological pathways. The most frequent NMI (ANKRD28, 28.5%; see also Example 7, which determined the frequency of an SV is much higher in the MS population) is a subunit of a phosphatase that regulates NF-κB, a signaling pathway that has been heavily associated with MS. Several current treatments are directly or indirectly linked to this molecule as well. The second most frequent NMI (26.7%) resides in the ST6GALNAC5 gene, which is involved in the biosynthesis of ganglioside GD1a from GM1b. Both of these gangliosides are increased in individuals with a type of MS called "relapsing remitting" during their first MS attack. As with ANKRD28, these two gangliosides are also in the NF-κB pathway. Exogenous addition of GD1a gangliosides have been shown to promote remyelination, which is dysfunctional in MS. The protein product of a third gene (SEMA3A, 11.3%), was shown to be lower in plasma from patients with MS and the PLXNA2 gene in this analysis (12.4%) is a receptor for SEMA3A. Three other genes (DLC1, DISC1, and ITGB3) are also part of the SEMA3A pathway and show NMI in greater than 10% of the individuals.

Although the present disclosure provides references to preferred embodiments, persons skilled in the art will recognize that changes may be made in form and detail without departing from the spirit and scope of the invention.

**Table 1. Seventeen SNPs positive in the BlocBuster analyses. Minor allele frequencies are given for the two study groups, the 1000 Genome samples, and the HapMap project. Group I is the test between males only (N=336), Group II consisted of a larger dataset (N=471) of males and females. Fourteen SNPs were identified in both data sets. All of the loci have a repeat element within 50bp but are rare (<1%) with the exception of POLE. SR = Simple Repeat, SC = Self Chain repeat. Nine of the seventeen loci have an NMI elsewhere in the gene and the VWA8 gene was shown to be differentially regulated in single-cell transcriptomics reports from ASD post mortem brain tissue.**

| **SNPID** | **Gene** | **Test Group** | **Chr** | **Risk Allele** | **SV +/-50bp** | **Disorder Association** | **Reference** | **Function** | **Miami ASD** | **Miami Father** | **Miami Mother** | **AGPC ASD** | **AGPC Father** | **AGPC Mother** | **1000G EUR** | **HapMap CEU** |
|---|---|---|---|---|---|---|---|---|---|---|---|---|---|---|---|---|
| | | | | | | | | | N=386 | N=381 | N=381 | N=1138 | N=1137 | N=1137 | N=503 | N=174 |
| rs209737 | EPHA8 | I, II | 01 | C | SR | None | None | Neuronal development | 1.00 | 1.00 | 1.00 | 1.00 | 1.00 | 1.00 | 0.23 | N/A** |
| rs3806208 | LRRC42 | I, II | 01 | A | LINE L2b | Autism | Sgado et al. 2013 | Unknown | 1.00 | 1.00 | 1.00 | 1.00 | 1.00 | 1.00 | 0.00 | 0.00 |
| rs13017584 | IL1R1/IL1RL2^{%} | I, II | 02 | C | INDEL | Autism | Bartnik et al. 2014 | Immune response | 1.00 | 1.00 | 1.00 | 1.00 | 1.00 | 1.00 | 0.04 | N/A** |
| rs12996740 | Intergenic | I, II | 02 | C | SR | None | None | Unknown | 1.00 | 1.00 | 1.00 | 1.00 | 1.00 | 1.00 | 0.13 | 0.11 |
| rs16852995 | CHST2 | I, II | 03 | A | LINE L2c | Autism | Hu et al. 2006 | Immune response | 1.00 | 1.00 | 1.00 | 1.00 | 1.00 | 1.00 | 0.00 | 0.00 |
| rs2148553 | HELLS^{%} | I, II | 10 | A | HAL1 repeat | Autism | Nishimura et al. 2007 | DNA rec-repair | 1.00 | 1.00 | 1.00 | 1.00 | 1.00 | 1.00 | 0.37 | 0.37 |
| rs7299820 | FMNL3 | I, II | 12 | T | SR | Autism | Marshall et al. 2008 | Cytoskeleton | 1.00 | 1.00 | 1.00 | 1.00 | 1.00 | 1.00 | 0.01 | 0.00 |
| rs12823039 | POLE^{%} | I, II | 12 | G | SR | FILS Syndrome | Schmid et al. 2012 | DNA rec-repair | 1.00 | 1.00 | 1.00 | 1.00 | 1.00 | 1.00 | 0.00 | N/A** |
| rs348238 | VWA8^{%} | I, II | 13 | A | SR | Autism | Anney et al. 2010 | Mitochondria | 1.00 | 1.00 | 1.00 | 1.00 | 1.00 | 1.00 | 0.00 | 0.00 |
| rs17115710 | LOC105370737/UBE3A^{%} | I, II | 15 | C | LINE L2c | Autism | Yi et al. 2015 | Proteosome | 1.00 | 1.00 | 1.00 | 1.00 | 1.00 | 1.00 | 0.12 | 0.23 |
| rs17196874 | MKL2^{%} | I, II | 16 | C | SC | Autism | Holt et al. 2010 | Serum Response Factor | 1.00 | 1.00 | 1.00 | 1.00 | 1.00 | 1.00 | 0.04 | 0.09 |
| rs11909948 | KCNJ6^{%} | I, II | 21 | A | LINE L2a | Lubinsky Syndrome | Masotti et al. 2015 | Neuronal development | 1.00 | 1.00 | 1.00 | 1.00 | 1.00 | 1.00 | 0.28 | 0.27 |
| rs28637143 | DCX | I, II | X | G | LINE L1M5 | Lissencephaly | Pilz et al. 1998 | Neuronal development | 1.00 | 1.00 | 1.00 | 1.00 | 1.00 | 1.00 | 0.00 | N/A** |
| rs9697856 | ESXI | I, II | X | C | SR | None | None | Placental development | 1.00 | 1.00 | 1.00 | 1.00 | 1.00 | 1.00 | 0.00 | N/A** |
| rs1152907 | IRAK3^{%} | II | 12 | C | INDEL | Asthma | Balaci et al. 2007 | Immune response | 1.00 | 1.00 | 1.00 | 1.00 | 1.00 | 1.00 | 0.40 | N/A** |
| rs260148 | PCDH9" | II | 13 | T | INDEL | Autism | Marshall et al. 2008 | Neuronal development | 0.41 | 0.40 | 0.40 | 0.41 | 0.39 | 0.41 | 0.38 | 0.65 |
| rs3803697 | WDR90 | II | 16 | C | SC | Autism | Egawa et al. 2015 | Highly expressed in brain | 1.00 | 1.00 | 1.00 | 1.00 | 1.00 | 1.00 | 0.38 | N/A** |

| | | | | | | | | | | | | | | | | |
|---|---|---|---|---|---|---|---|---|---|---|---|---|---|---|---|---|
| * Mendel test not performed X-chromosome ** Data unavailable % At least one NMI in the gene | | | | | | | | | | | | | | | | |

## Claims

1. A method of identifying at least one inherited or *de novo* structural variation in a population of genomes from individuals as a diagnostic marker for a disease or disorder, the method comprising:
assembling single nucleotide polymorphism (SNP) data from parents and their offspring;
analyzing the SNP data for at least one non-Mendelian inheritance pattern (NMI),
wherein the NMI is a potential structural variation;
scoring the NMI to identify large structural variations from sequential SNPs that demonstrate NMI in the offspring;
removing SNPs that demonstrate NMI in the offspring but that overlap with known existing variation;
identifying conserved regions of the genome to filter regions that should be conserved but include a structural variation; and
screening the NMI for potentially biologically important inherited or *de novo* structural variation.

2. The method of claim 1, comprising verifying NMI of high frequency in a population to determine if the NMI of high frequency are structural variations, preferably wherein NMI of high frequency are present at greater than 5% in the population.

3. The method of claim 2, comprising removing SNPs of NMI that are structural variations but that overlap with known existing variation.

4. The method of any one of claims 1 to 3, wherein the potentially biologically important structural variation is selected from one or more of a structural variation that resides in a gene in which less than 5% of normal individuals have a known structural variation; there is a custom correlation coefficient bloc in the gene; a frequency of the NMI at one site in the gene is greater than 5% in a diseased population; and there is a run of at least four SNPs with NMI in a row.

5. The method of any one of claims 1 to 4, wherein identifying conserved regions of the genome is performed by a custom correlation coefficient (CCC) analysis.

6. The method of any one of claims 1 to 5, comprising assigning a probability on having a run of NMI and maintaining SNPs with a run of NMI greater than 4.

7. The method of any one of claims 1 to 6, comprising removing NMI attributable to high levels of masked repetitive elements that exceeded an expected frequency as determined by a Chi-Square test.

8. The method of any of claim 1 to 7, comprising identifying locations of the structural variations and identifying locations of conserved blocs of genetic information, and further comprising using the locations of the structural variations and the locations of the conserved blocs of genetic information to identify locations of rare structural variations in genes that have conserved blocs of genetic information.

9. A method of identifying a structural variation in the genome of a patient, wherein a disease or disorder is related to the structural variation, the method comprising:
assembling single nucleotide polymorphism (SNP) data of the patient and their parents;
analyzing the SNP data for at least one non-Mendelian inheritance pattern (NMI),
wherein the NMI is a potential structural variation;
scoring the NMI to identify large structural variations from sequential SNPs that demonstrate NMI in the patient;
removing as false positives SNPs that demonstrate NMI in the offspring but that overlap with known existing variation;
identifying conserved regions of the genome to filter regions that should be conserved but include a structural variation; and
screening the NMI for structural variation related to the disease or disorder.

10. The method of claim 9, comprising verifying NMI of high frequency in a population to determine if the NMI of high frequency are structural variations, preferably wherein NMI of high frequency are present at greater than 5% in the population.

11. The method of claim 10, comprising removing SNPs of NMI that are structural variations but that overlap with known existing variation.

12. The method of any one of claims 9 to 11, wherein the structural variation related to the disease or disorder is selected from one or more of a structural variation that resides in a gene in which less than 5% of normal individuals have a known structural variation; there is a custom correlation coefficient bloc in the gene; a frequency of the NMI at one site in the gene is greater than 5% in a diseased population; and there is a run of at least four SNPs with NMI in a row.

13. The method of any one of claims 9 to 12, wherein identifying conserved regions of the genome is performed by a custom correlation coefficient (CCC) analysis.

14. The method of any one of claims 9 to 13, comprising filtering out of structural variations that are not specific to the disease or disorder.

15. The method of any one of claims 9 to 14, wherein the disease or disorder is to be treated and the intended treatment includes a gene editing technology, preferably wherein the gene editing technology includes CRISPR, or the intended treatment includes administration of CAR T cells.
